# EUROPEAN PATENT APPLICATION

(11) **EP 4 595 941 A1**
(43) Date of publication of application: **06.08.2025**
(21) Application number: 23871048.7
(22) Date of filing: 28.09.2023
(51) Int. Cl.: A61H 23/00

(54) **CHARGING SYSTEM, ELECTRICAL ISOLATION SYSTEM, CONTROL SYSTEM, AND SHOCKWAVE DEVICE**

(30) Priority: 30.09.2022 CN 202211215145; 30.09.2022 CN 202211214773; 30.09.2022 CN 202222613809 U
(71) Applicant: SMARTWAVE MEDICAL HK LIMITED, Queensway, Admiralty (HK)
(72) Inventor: DING, Shangshang, Suzhou, Jiangsu 215000 (CN); CHEN, Jianfeng, Suzhou, Jiangsu 215000 (CN); ZHANG, Yi, Suzhou, Jiangsu 215000 (CN)
(74) Representative: Isern Patentes y Marcas S.L.
(86) International application number: PCT/CN2023/122711
(87) International publication number: WO 2024/067823

(57) **Abstract**

Provided are a charging system, an electrical isolation system, a control system, and a shockwave device. The charging system is provided with at least two electrical isolation circuits, thereby improving the frequency of sending charging control signals and further improving the frequency of charging a shockwave generation apparatus. The electrical isolation system can greatly improve the dielectric strength of the electrical isolation system, reduce a leakage current on the surface of a shockwave generator, and ensure the safety performance of the shockwave generator. The shockwave device can effectively monitor the discharge energy of a shockwave emitter, thereby improving treatment safety, controllability, and working efficiency.

## Description

### Technical field

This application relates to the technical field of medical instruments, and in particular, to a charging system, an electrical isolation system, a control system, and a shock wave device.

### Background

The shock wave device for treating heart valve and/or vascular calcification is an active medical device directly applied to the heart, and the working voltage of the active medical device is as high as 10 kV, and its electrical safety classification is CF-type, which means that, the leakage current of the patient allowed in the normal state is less than or equal to 0.01 mA, but the current leakage current of the patient applied to the heart or blood vessel in the prior art is less than or equal to 0.01 mA, but the leakage current of the patient in the normal state is less than or equal to 0.1 mA; Therefore, there is a need for an improved electrical isolation system to ensure that the operating voltage of the shock wave device is up to above 10 kV and the patient leakage current allowed in the normal state conforms to the CF type safety performance criteria.

At the same time, the existing shock wave generating device applied to heart valve and vascular calcification has a long charging time and a low energy storage voltage, basically can only output a pulse high voltage of about 3 kV and a frequency of 1 Hz, and therefore is only suitable for the situation that the distance between the electrode and the to-be-impacted position is relatively close, and when the distance between the electrode and the to-be-impacted position is relatively long, the energy of the shock wave can be rapidly attenuated in the transmission, and meanwhile, the frequency of the existing shock wave generating device cannot be adjusted, so that the existing shock wave generating device cannot be applied to different scenes. In addition, in the treatment process of treating the heart valve and/or the vascular calcification by using the shock wave device, the shock wave emitter entering the human body needs to be electrically connected with the shock wave generator outside the body, and the shock wave emitter receives the voltage or current sent by the extracorporeal shock wave generator and releases the shock wave, however, the existing in-vivo shock wave device cannot detect the energy output condition of the shock wave transmitter, and the safety and controllability are poor.

Based on the shortcomings of the prior art, there is an urgent need to provide a charging system, an electrical isolation system, a control system, and a shock wave device to solve the above problems.

### Summary

In order to solve the above technical problem, the present application provides a charging system, an electrical isolation system, a control system, and a shock wave device.

A first aspect of the present application sets forth a charging system, comprising a charging control module and a charging power supply module connected in parallel;
the charging control module comprises a power controller and at least two parallel electrical isolation circuits; the charging power supply module comprises a high-frequency conversion module and a rectification module which are connected in series;
an input end of the power controller is configured to receive an initial charging modulation signal, an output end of the power controller is electrically connected to an input end of the electrical isolation circuit, and an output end of the electrical isolation circuit is electrically connected to a signal input end of the high-frequency conversion module;
a power input terminal of the high-frequency conversion module is electrically connected to a charging power supply, and an output terminal of the high-frequency conversion module is electrically connected to the rectification module;
the initial charging modulation signal generates a charging control signal after passing through the power controller, and is transmitted to the high-frequency conversion module through at least two electrical isolation circuits, and the high-frequency conversion module is turned on in response to the charging control signal to charge a charging capacitor of the discharging energy storage module.

Further, the power controller is configured to perform frequency raising processing on the initial charging modulation signal, where a signal frequency of the charging control signal is higher than the initial charging modulation signal.

Further, the charging control signal comprises at least two charging control sub-signals of different phases, and at least two of the charging control sub-signals are transmitted to the high-frequency conversion module through the at least two electrical isolation circuits respectively;
the high-frequency conversion module is configured to perform frequency raising processing on the at least two charging control sub-signals to output at least two target control signals, where a signal frequency of the target control signal is higher than the charging control sub-signal.

Further, each of the at least two electrical isolation circuits comprises two electrical isolation branches connected in parallel;
the high-frequency conversion module comprises at least four signal input ends, and the electrical isolation branches are arranged in one-to-one correspondence with the signal input ends.

Further, the electrical isolation branch comprises an isolation unit, a switch circuit, and an independent power supply, an input end of the isolation unit is electrically connected to the power controller, and the switch circuit is electrically connected to the isolation unit, the independent power supply, and the high-frequency conversion module.

Further, the charging power supply module further comprises a first voltage transformation device, the first voltage transformation device is connected in series with the high-frequency conversion module and the rectification module, and an input end voltage of the first voltage transformation device is less than an output end voltage of the first voltage transformation device.

Further, the charging control module further comprises a first charging isolation circuit connected in series with the power controller, and the first charging isolation circuit is configured to receive the initial charging modulation signal and transmit the initial charging modulation signal to the power controller.

Further, the charging control module further comprises a voltage acquisition circuit;
the voltage acquisition circuit is configured to be electrically connected to the charging capacitor, and the voltage acquisition circuit is configured to detect an energy storage voltage of the charging capacitor and output a voltage feedback signal based on the energy storage voltage.

Further, the charging power supply module further comprises an isolation power supply, and the charging power supply, the isolation power supply, and the high-frequency conversion module are sequentially connected in series.

A second aspect of the present application further sets forth an electrical isolation system, which is applied to a shock wave device, and the electrical isolation system comprises: a signal trigger module, a first rectification module, a high-frequency conversion circuit, and a discharge energy storage module which are sequentially connected in series;
an input terminal of the first rectification module is connected to a power supply, and is configured to convert an AC current into a DC current, and transmit the DC current to the high-frequency conversion circuit; and
the high-frequency conversion circuit comprises a high-frequency conversion module and an electrical isolation branch; the high-frequency conversion module is configured to convert the direct current into a high-frequency current, and transmit the high-frequency current to the discharge energy storage module; the high-frequency conversion module comprises a plurality of high-frequency conversion units, the electrical isolation branch comprises N isolation units, and the high-frequency conversion units are connected to the first controller through the isolation unit; where N is an even number greater than 2;
an output terminal of the discharge energy storage module is connected to the shock wave transmitter;
an output end of the signal trigger module is connected to the discharge energy storage module, and the signal trigger module is configured to control the discharge energy storage module to supply power to the shock wave transmitter.

Further, one of the isolation units is connected to at least one of the high-frequency conversion units, and preferably, one of the isolation units is connected to one of the high-frequency conversion units.

Further, the electrical isolation branch comprises N independent power supplies;

The N independent power sources are connected to the N isolation units in a one-to-one correspondence.

Further, the signal trigger module comprises an optocoupler and a pulse switch circuit;
the optocoupler is connected to the pulse switch circuit, the pulse switch circuit is connected to the discharge energy storage module, and the optocoupler controls the pulse switch circuit to be turned on based on a received preset trigger signal, so that the discharge energy storage module provides electric energy to the shock wave transmitter.

Further, the discharge energy storage module comprises a charging capacitor and a discharge control module;
a charging end of the charging capacitor is connected to the high-frequency conversion circuit, and a discharging end of the charging capacitor is connected to the shock wave transmitter through the discharge control module.

A third aspect of the present application further sets forth a control system applied to a shock wave device, comprising: a signal triggering module, a charging control module, a charging module, and a control feedback module;
the charging control module is electrically connected to the charging module;
the charging module comprises a high-frequency conversion module and a discharge energy storage module which are connected in series, and a signal input end of the high-frequency conversion module is electrically connected to an output end of the charging control module;
the control feedback module is electrically connected to the charging control module and the discharge energy storage module, respectively;
a power input end of the high-frequency conversion module is electrically connected to a power supply, an output end of the high-frequency conversion module is electrically connected to the discharge energy storage module, and the high-frequency conversion module can charge the discharge energy storage module;
an output end of the signal trigger module is electrically connected to the discharge energy storage module, an output end of the discharge energy storage module is electrically connected to the shock wave transmitter, and a charging trigger signal output by the signal trigger module can be transmitted to the discharge energy storage module, so that the discharge energy storage module and the shock wave transmitter are in an on state.

Further, the charging control module comprises a power controller and at least two electrical isolation circuits connected in parallel; an input end of the power controller can receive an initial charging modulation signal, an output end of the power controller is connected to an input end of the electrical isolation circuit, and an output end of the electrical isolation circuit is electrically connected to a signal input end of the high-frequency conversion module.

Further, each of the at least two electrical isolation circuits comprises two electrical isolation branches connected in parallel;
the high-frequency conversion module comprises at least four signal input ends, and the electrical isolation branches are arranged in one-to-one correspondence with the signal input ends.

Further, the charging module comprises a first voltage transformation device, the high-frequency conversion module is connected with the discharge energy storage module through the first voltage transformation device, and an input end voltage of the first voltage transformation device is smaller than an output end voltage of the first voltage transformation device.

Further, the electrical isolation branch comprises an isolation unit, a switch circuit, and an independent power supply;
the switch circuit is connected to the power controller through the isolation unit, a power connection end of the switch circuit is connected to the independent power supply, and an output end of the switch circuit is connected to a signal input end of the high-frequency conversion module.

Further, the discharge energy storage module comprises a charging unit, a charging capacitor, and a discharge control module;
an input end of the charging unit is electrically connected to the high-frequency conversion module, an output end of the charging unit is electrically connected to a charging end of the charging capacitor, and a discharging end of the charging capacitor is electrically connected to the shock wave generator through the discharge control module.

Further, the control feedback module comprises a transmitter sensing device, a transmitter monitoring module, and a trigger signal monitoring module;
the transmitter sensing device is disposed opposite to the shock wave transmitter, the shock wave transmitter is electrically connected to the shock wave generator, and the transmitter monitoring module is electrically connected to the transmitter sensing device and the discharge energy storage module, respectively;

The trigger signal monitoring module is electrically connected to the charging capacitor.

Further, the control feedback module further comprises a voltage adjustment module, an adjustment voltage monitoring module, a charging voltage monitoring module, and a first voltage comparison module, wherein the first voltage comparison module is electrically connected to the adjustment voltage monitoring module and the charging voltage monitoring module, respectively;
the adjustment voltage monitoring module is configured to detect a voltage setting signal output by the voltage adjustment module, and transmit the voltage setting signal to the first voltage comparison module; the charging voltage monitoring module is configured to detect a current voltage signal of the charging capacitor and transmit the current voltage signal to the first voltage comparison module; and the first voltage comparison module is configured to compare the voltage setting signal with the current voltage signal to generate a voltage comparison feedback signal, where the voltage comparison feedback signal is configured to indicate an on-off state between the charging unit and the charging capacitor.

Further, the control feedback module further comprises a second voltage comparison module, and the second voltage comparison module is electrically connected to the adjustment voltage monitoring module;
the second voltage comparison module is configured to receive the voltage setting signal transmitted by the adjustment voltage monitoring module, and compare the voltage setting signal with an output voltage threshold to generate an output voltage feedback signal, where the output voltage feedback signal is configured to indicate an on-off state between the charging unit and the charging capacitor.

Further, the control feedback module further comprises a temperature monitoring module and a sensing device disposed on the shock wave generator, and the sensing device is electrically connected to the temperature monitoring module;
the sensing device is configured to collect a working temperature of a target component in the shock wave generator, and transmit the working temperature to the temperature monitoring module; and
the temperature monitoring module is configured to generate a temperature feedback signal based on the working temperature and a preset working temperature threshold, where the temperature feedback signal is configured to indicate an on-off state between the charging unit and the charging capacitor.

A fourth aspect of the present application further sets forth a shock wave device, comprising a shock wave transmitter and the control system as described above.

The embodiment of the embodiments of the present application has the following beneficial effects.
1. In the present application, the charging control module and the charging power supply module are arranged at the same time, and the charging power supply module charges the charging capacitor only when receiving the charging control signal sent by the charging control module, so that the charging control module and the charging power supply module cooperate with each other to achieve the effect of cooperatively controlling the charging control module and the charging power supply module; At the same time, by providing at least two electrical isolation circuits, the frequency of the charging control module sending the charging control signal to the charging power supply module is improved, thereby improving the charging frequency of the charging power supply module to the charging capacitor, and further improving the shock wave transmission frequency.
2. By setting the signal trigger circuit and the first rectifying circuit, the high-frequency conversion circuit and the discharge energy storage module which are sequentially connected in series, power supply to the shock wave transmitter is achieved, specifically, the input end of the first rectifying circuit is connected to the power supply, and is configured to convert the alternating current into a direct current and deliver the direct current to the high-frequency conversion circuit; The high-frequency conversion module is configured to convert a direct current into a high-frequency current and deliver a high-frequency current to the discharge energy storage module The high-frequency conversion module comprises a plurality of high-frequency conversion units, the first isolation module comprises n electrical isolation branches, and the high-frequency conversion units are connected to the power controller through electrical isolation branches; n is an even number greater than 2; and an output end of the discharge energy storage module is connected to the shock wave transmitter. The technical solution provided by the present application can greatly improve the dielectric strength of the electrical isolation system, reduce the leakage current on the surface of the shock wave transmitter, and ensure the safety performance of the shock wave transmitter.
3. By arranging the transmitter sensing device and the transmitter monitoring module at the shock wave transmitter, the target state parameter of the shock wave transmitter in the working state can be monitored in real time, and whether the generator negative feedback signal is output to the shock wave generator or not is judged based on the target state parameter and the preset discharge condition, so that real-time monitoring and feedback of the energy output of the shock wave transmitter are realized, and the charging state of the shock wave generator is controlled in time, so that the system safety and controllability are remarkably improved.

### Brief description of the drawings

In order to more clearly illustrate the technical solutions of the present disclosure, the following briefly describes the accompanying drawings that need to be configured in the description of the embodiments or the prior art. Obviously, the accompanying drawings in the following description are merely some embodiments of this application, and for a person of ordinary skill in the art, other drawings may be obtained based on these accompanying drawings without creative efforts.
FIG. 1 is a structural diagram of a charging system according to Embodiment 1 of this application;
FIG. 2 is a detailed structural diagram of a charging system according to Embodiment 1 of this application;
FIG. 3 is a structural diagram of a high-frequency conversion module according to Embodiment 1 of this application;
FIG. 4 is a partial structural diagram of a charging system according to Embodiment 1 of this application;
FIG. 5 is a structural diagram of an electrical isolation system for a shock wave device according to Embodiment 2 of the present application;
FIG. 6 is a structural diagram of a high-frequency conversion circuit according to Embodiment 2 of this application;
FIG. 7 is a structural diagram of a discharge control module according to Embodiment 2 of the present application;
FIG. 8 is a structural diagram of a shock wave transmitter according to Embodiment 2 of the present application;
FIG. 9 is a schematic structural diagram of a control system for a shock wave device according to Embodiment 3 of this application;
FIG. 10 is a schematic structural diagram of a charging control module according to Embodiment 3 of this application;
FIG. 11 is a schematic structural diagram of a discharge control module according to Embodiment 3 of this application;
FIG. 12 is a schematic structural diagram of a shock wave apparatus according to Embodiment 3 of this application;
FIG. 13 is a schematic diagram of a partial structure of a shock wave apparatus according to Embodiment 3 of this application;
FIG. 14 is a cross-sectional view of a balloon in a shock wave device according to Embodiment 3 of the present application;
FIG. 15 is a schematic structural diagram of a control feedback module according to Embodiment 3 of this application.

Reference numbers:
100-Charging Control Module; 101-first charging isolation circuit; 102-Power Controller; 103-electrical isolation branch; 104-isolation unit; 105-switch circuit; 106-Independent Power Supply; 200-charging power supply module; 201-isolated power supply; 202-high frequency conversion module; 203-First voltage transformation device; 204-rectification module; 205-charging power supply; 206-first isolation module; 207-second rectifying circuit; 208-optocoupler; 209-thyristor; 210-Second Voltage Transformation Device; 211-pulse switching circuit; 212-first voltage output end; 213-second voltage output end; 214-power supply; 215-first rectifying circuit; 216-high frequency conversion circuit; 217-high frequency conversion unit; 218-Discharge Energy Storage Module; 219-discharge control module; 220-discharge control unit; 221-signal isolation circuit; 222-electrical isolation circuit; 223-high voltage isolation circuit; 224-shock wave emitter; 225-isolation transformer; 226-first controller; 227-first rectification module; 228-electrode; 229-Second rectification module; 230-charging module; 231-Charging Capacitor; 232 charging unit; 234-electrical isolation sub-circuit; 300-signal triggering module; 400-control feedback module; 500-positioning inner tube; 501-emitter induction device; 502-balloon; 503-regulation voltage monitoring module; 504-charging voltage monitoring module; 505-first voltage comparison module; 506-voltage regulation module; 507-second voltage comparison module; 508-temperature monitoring module; 509-induction device; 510-emitter monitoring module; 511-trigger signal monitoring module; 512-start signal monitoring module; 513-threshold voltage regulation module.

### Detailed description

The following clearly and completely describes the technical solutions in the embodiments of this application with reference to the accompanying drawings in the embodiments of this application. All other embodiments obtained by a person of ordinary skill in the art based on the embodiments of this application without creative efforts shall fall within the protection scope of this application.

It should be noted that the terms "first", "second", and the like in the specification, claims, and accompanying drawings of this application are configured to distinguish similar objects, and are not necessarily configured to describe a specific order or sequence. It should be understood that the data configured in this way can be interchanged under appropriate circumstances, so that the embodiments of the present application described herein can be implemented in an order other than those illustrated or described herein. In addition, the terms "comprise" and "have" and any deformation thereof are intended to cover a non-exclusive inclusion, for example, a process, method, system, product, or device that comprises a series of steps or units is not necessarily limited to those steps or units listed clearly, but may comprise other steps or units that are not clearly listed or inherent to these processes, methods, products, or devices.

### Embodiment 1

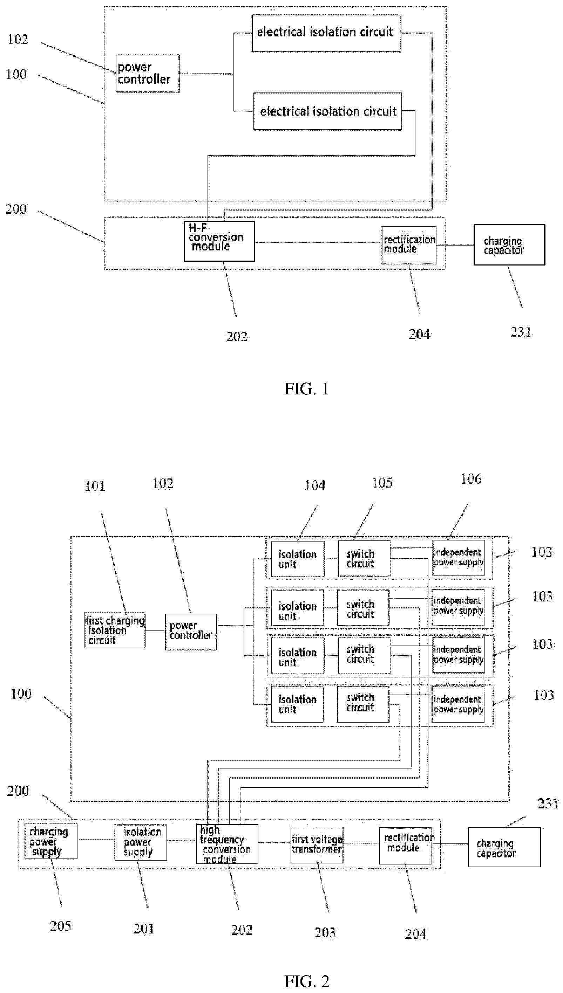

Referring to FIG. 1 to FIG. 4, this embodiment provides a charging system, comprising a charging control module 100 and a charging power supply module 200 connected in parallel; the charging control module 100 comprises a power controller 102 and at least two electrical isolation circuits connected in parallel; the charging power supply module 200 comprises a high-frequency conversion module 202 and a rectification module 204 connected in series; an input end of the power controller 102 is configured to receive an initial charging modulation signal, an output end of the power controller 102 is electrically connected to an input end of the electrical isolation circuit, and an output end of the electrical isolation circuit is electrically connected to a signal input end of the high-frequency conversion module 202; The power input end of the high-frequency conversion module 202 is electrically connected to the charging power supply, and the output end of the high-frequency conversion module 202 is electrically connected to the rectification module 204; and the initial charging modulation signal generates a charging control signal after passing through the power controller 102, and is transmitted to the high-frequency conversion module 202 through the at least two electrical isolation circuits, and the high-frequency conversion module 202 is turned on in response to the charging control signal to charge the charging capacitor 231.

An existing shock wave generating device can be configured for treating heart valve and vascular calcification, the shock wave generating device of the existing shock wave generating device is long in charging time, the energy storage voltage is low, only about 3 kV and 1 Hz pulse high voltage can be output basically, so that enough shock wave energy can be obtained only when the shock wave generating point is close to the target position in practical application, and only when the distance between the electrode and the to-be-impacted position is relatively close, when the distance between the electrode and the to-be-impacted position is relatively long, the energy of the shock wave can be rapidly attenuated in the transmission; Meanwhile, because the frequency of the shock wave generating device is not adjustable, the existing shock wave generating device cannot be applied to different scenes, which affects the adaptability of the existing shock wave generating device.

It should be noted that the charging control module 100 and the charging power supply module 200 are provided at the same time in this embodiment, and the charging power supply module 200 charges the charging capacitor 231 only when receiving the charging control signal sent by the charging control module 100, so that the charging control module 100 and the charging power supply module 200 cooperate with each other to achieve the effect of cooperatively controlling the charging control module 100 and the charging power supply module 200; at the same time, by providing at least two electrical isolation circuits, the frequency of sending the charging control signal by the charging control module 100 to the charging power supply module 200 is improved, thereby improving the charging frequency of the charging power supply module 200 to the charging capacitor 231, thereby improving the frequency of the shock wave generation.

It should also be noted that when the high-frequency conversion module 202 receives the charging control signal to be turned on, the high-frequency conversion module 202 performs frequency rising processing on the charging control signal to obtain at least two upconverted charging control sub-signals, and charges the charging capacitor 231 based on the rectified voltage and the at least two paths of charging control sub-signals, so that the charging capacitor 231 operates at a high voltage and a high frequency, and the charging capacitor 231 releases more shock wave energy in unit time.

In this embodiment, the leakage current of the charging capacitor 231 in the normal working state may be 0.002 mA -0.006 mA, and the dielectric strength of the charging system may be 15 kV.

In some possible embodiments, the power controller 102 is configured to perform frequency raising processing on the initial charging modulation signal, where a signal frequency of the charging control signal is higher than an initial charging modulation signal.

Specifically, the power controller 102 is a PWM controller, and the initial charging modulation signal is a signal sent by the microcontroller; and the PWM controller performs frequency rising processing on the initial charging modulation signal to obtain a charging control signal, so as to greatly enhance the capability of the charging system to drive the load. In an embodiment, the PWM controller may output at least two different phase charging control signals, the phase difference may be 180°, and the charging control signal may be a square wave signal.

In an embodiment, the power controller 102 comprises a control chip and a configuration circuit thereof, the initial modulation signal is connected with the soft start pin of the chip, when the current of the initial charge modulation signal is too large, the control chip stops oscillating instantly, the effective current is reduced, the load overcurrent is prevented from damaging the power controller 102, meanwhile, the configuration circuit at least comprises an oscillator trigger, the oscillator generates sawtooth wave oscillation through the external time base capacitor and the resistor, meanwhile, a clock pulse signal is generated, and the pulse width of the signal corresponds to the falling edge of the sawtooth wave. The clock pulse serves as a trigger signal for the phase splitter composed of flip-flops to generate a square wave signal having a phase difference of 180°.

Specifically, the control chip may be an SG 3523.

More specifically, since the output capability of the microcontroller is limited, a square wave signal with an excessively high frequency cannot be directly generated, and the square wave signal is further amplified by the power controller 102, so as to greatly enhance the capability of the charging system to drive the load.

In some possible embodiments, the charging control signal comprises at least two charging control sub-signals of different phases, and each charging control sub-signal is transmitted to the high-frequency conversion module 202 through the at least two electrical isolation branches 103 respectively; and the high-frequency conversion module 202 is configured to perform frequency rising processing on the at least two charging control sub-signals to output at least two paths of target control signals, where a signal frequency of the target control signal is higher than a charging control sub-signal.

It should be noted that, in this embodiment, the power controller 102 performs primary frequency amplification on the initial charging modulation signal to obtain a charging control signal; and the charging control signal performs secondary frequency amplification on the initial charging modulation signal by using at least two electrical isolation circuits to obtain a target control signal, that is, sequentially performs multiple times of frequency rising on the signal, so that the charging capacitor 231 can be charged at a high frequency in this embodiment.

In some embodiments, the charging power supply module 200 further comprises a first voltage transformation device 203, the first voltage transformation device 203 is connected in series with the high-frequency conversion module 202 and the rectification module 204, and an input end voltage of the first voltage transformation device 203 is less than an output end voltage of the first voltage transformation device 203; when the charging frequency is improved, the output voltage of the first voltage transformation device 203 is set to perform boost processing, so that the charging voltage of the charging power supply module 200 to the charging capacitor 231 is improved, and the charging capacitor 231 releases more shock wave energy per unit time.

In some possible embodiments, each of the at least two electrical isolation circuits comprises two electrical isolation branches 103 connected in parallel; the high-frequency conversion module 202 comprises at least four signal input ends, and the electrical isolation branches 103 are arranged in one-to-one correspondence with the signal input ends; by controlling the high-frequency conversion module 202 through the plurality of electrical isolation branches 103, the leakage current in the charging process of the charging capacitor 231 is effectively reduced, so that it is guaranteed that the charging capacitor 231 can be directly applied to the heart, the dielectric strength of the charging system is greatly improved, the safety of the whole charging system under a high working voltage is ensured, and it is ensured that the charging capacitor 231 does not negatively affect the human body.

The at least two charging control sub-signals output at least four charging control sub-signals after passing through the at least two electrical isolation circuits; and the high-frequency conversion module 202 is configured to respectively receive at least four charging control sub-signals based on at least four signal input ends, and perform pairwise combination processing on the at least four charging control sub-signals to output at least two paths of target control signals.

In some possible embodiments, the high-frequency conversion module 202 comprises a first voltage output terminal 212, a second voltage output terminal 213, and at least two signal receiving circuits; the first voltage output end 212 and the second voltage output end 213 are both electrically connected to the first voltage transformation device 203, and the first voltage output end 212 and the second voltage output end 213 are configured to supply power to the first voltage transformation device 203; the number of signal receiving circuits corresponds to the number of electrical isolation circuits, each signal receiving circuit is configured to receive two charging control sub-signals, and control the first voltage output end 212 and the second voltage output end 213 to supply power to the first voltage transformation device 203 after receiving the two charging control sub-signals.

It should be noted that, in this embodiment, at least two charging control sub-signals are sent to the high-frequency conversion module 202, and are processed by at least two signal receiving circuits in the high-frequency conversion module 202 to obtain at least two target control signals; a frequency of charging the charging capacitor 231 based on the at least two target control signals within a unit time is significantly higher than a frequency of performing charging processing on the charging capacitor 231 based on a control signal in a unit time in the prior art; that is, after the at least four charging control sub-signals are processed by the signal receiving circuit, the at least two target control signals are obtained to realize the second-level frequency rising, thereby realizing high-frequency charging of the charging capacitor 231 per unit time, and ensuring that the charging capacitor 231 releases more shock wave energy per unit time.

Specifically, the signal receiving circuit comprises a high-frequency conversion unit and a high-frequency conversion unit, the high-frequency conversion unit is configured to receive one charging control sub-signal, the high-frequency conversion unit is configured to receive another charging control sub-signal, and the two charging control sub-signals belong to an in-phase signal.

More recently, when the high-frequency conversion unit receives one charging control sub-signal, and the high-frequency conversion unit receives another charging control sub-signal, the first voltage output terminal 212 and the second voltage output terminal 213 supply power to the first voltage transformation device 203.

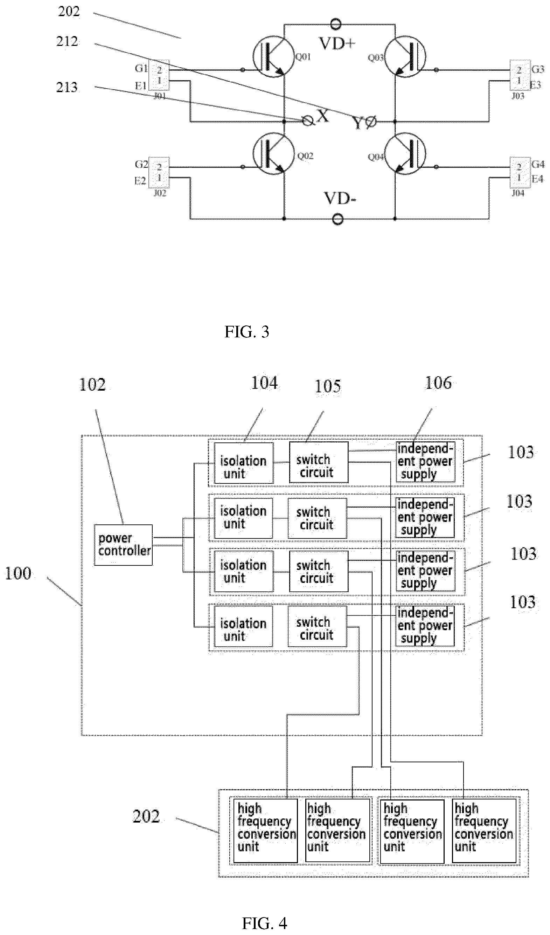

Specifically, referring to FIG. 3 and FIG. 4, when the high-frequency conversion module 202 comprises two signal receiving circuits, the high-frequency conversion unit of one signal receiving circuit corresponds to Q01, and the high-frequency conversion unit corresponds to Q04; the high-frequency conversion unit of the other signal receiving circuit corresponds to Q02, and the high-frequency conversion unit corresponds to Q03; When one signal receiving circuit turns on the square wave, Q01 and Q04 are turned on, X = VD +, Y = VD -, and XY is a forward square wave; when the other signal receiving circuit turns on the output square wave, Q02 and Q03 are turned on, X = VD -, Y = VD +, and XY is a negative square wave, it can be seen that the charging control signal is amplified again through the IGBT module signal to obtain the target control signal, and charging of the charging capacitor 231 is directly controlled.

In some possible embodiments, referring to FIG. 2, when the number of the electrical isolation circuits is two, the number of the signal receiving circuits is also two, the two signal receiving circuits respectively receive the two charging control sub-signals, and after receiving the corresponding charging control sub-signals, the two signal receiving circuits sequentially supply power to the first voltage transformation device 203; at the same time, the frequency of the shock wave of the charging capacitor 231 can be adjusted, it can also be ensured that the leakage current of the whole charging system is within the safety range, that is, the leakage current of the charging capacitor 231 in the normal working state is controlled to be 0.002 mA -0.006 mA, so that the dielectric strength of the whole charging system can be controlled at 15 kV.

In some other embodiments, when the number of the electrical isolation circuits is three, and the number of the signal receiving circuits is also three, the three signal receiving circuits respectively receive the two charging control sub-signals, and after receiving the corresponding charging control sub-signals, the three-way signal receiving circuit sequentially supplies power to the first voltage transformation device 203; when the electrical isolation circuit is three paths, the frequency of the shock wave released by the charging capacitor 231 in unit time is higher than that of the electrical isolation circuit, that is, the more the number of electrical isolation circuits is in a reasonable range, the charging capacitor 231 releases more shock wave energy per unit time.

For example, when the conduction quantity of the electrical isolation circuit is two, the charging frequency may reach 30 Hz, and when the conduction quantity of the electrical isolation circuit is three, the charging frequency may reach 50 Hz, and by analogy, it should be noted that when the charging frequency increases, the volume of the corresponding charging system may be increased, thereby increasing the leakage current of the entire charging system. Therefore, the number of conduction of the electrical isolation circuit is not limited in this embodiment.

For example, the normal operating voltage of the charging capacitor 231 may be greater than or equal to 10 kV, thereby reducing the leakage current on the surface of the charging capacitor 231 having a working voltage greater than or equal to 10 kV, so as to ensure that the charging capacitor 231 may be directly applied to the heart valve, etc. greatly improving the dielectric strength of the charging system, ensuring the safety of the entire charging system at a high operating voltage, and ensuring that the charging capacitor 231 does not negatively affect the human body.

In some embodiments, the electrical isolation branch 103 comprises an isolation unit 104, a switch circuit 1052, and an independent power supply 106, an input end of the isolation unit 104 is electrically connected to the power controller 102, and the switch circuit 1052 is electrically connected to the isolation unit 104, the independent power supply 106, and the high-frequency conversion module 202; by using the respective corresponding independent power supply 106 to each electrical isolation branch 103, the leakage current released by the isolation unit 104 can be effectively reduced, and the effective control of the high-frequency conversion module 202 can be ensured; and if one independent power supply 106 is configured to supply power to the multi-channel electrical isolation branch 103 at the same time, the leakage current of the electrical isolation circuit can be greatly increased, so that the stability of the charging system in the charging process cannot be ensured, and the safety performance of the charging capacitor 231 when releasing the shock wave is affected.

Specifically, the switch circuit 1052 is configured to isolate the isolation unit 104 from the independent power supply 106, and when a fault occurs in the isolation unit 104, cut off the current switch circuit 1052 in time to avoid affecting the independent power supply 106 or other electrical isolation branches 103; when the independent power supply 106 fails, the current switch circuit 1052 is cut off in time to avoid affecting the isolation unit 104 or other electrical isolation branches 103; meanwhile, each of the electrical isolation branches 103 is provided with the independent power supply 106 to ensure that the electrical isolation branches 103 do not affect each other, ensure the operation stability of the charging system, and the switch circuit 1052 is also configured to electrically isolate the electrical isolation branch 103 from the back-end circuit, thereby avoiding interference of the high-voltage high frequency to the front end, and further ensuring the operation stability of the charging system.

For example, when the two parallel electrical isolation branches 103 in the electrical isolation circuit are turned on under the control of the switching circuit 1052, the two corresponding signal receiving circuits in the high-frequency conversion module 202 electrically connected to the two parallel electrical isolation branches 103 are turned on to generate a target control signal, and the charging capacitor 231 is directly controlled to be charged; the on or off of the signal receiving circuit in the high-frequency conversion module 202 can be controlled by turning on or off the switch circuit 1052, thereby adjusting the charging frequency of the charging capacitor 231 and improving the adaptability of the charging capacitor 231.

Further, the isolation unit 104 comprises an optocoupler, an MOS transistor and a triode, the power supply configured by the part of components is an independent power supply, the output signal of the power generator 12 is electrically isolated from the rear end of the power generator 12 through the optocoupler, the MOS transistor and the triode, the interference of the high-voltage high-frequency part to the front end is avoided, and the operation stability of the charging system is improved.

In some other embodiments, each electrical isolation circuit in the at least two electrical isolation circuits comprises at least two electrical isolation branches 103 connected in parallel; the high-frequency conversion module 202 comprises at least four signal input ends, and the electrical isolation branches 103 are disposed in one-to-one correspondence with the signal input ends.

Specifically, each electrical isolation circuit comprises three electrical isolation branches 103 connected in parallel, and each electrical isolation circuit comprises two electrical isolation branches 103 connected in parallel to ensure that there are two electrical isolation branches 103 in each electrical isolation circuit 103 to remain in a closed state, which avoids that when the isolation unit 104 or the independent power supply 106 in one electrical isolation branch 103 fails, it is impossible to ensure that the three parallel electrical isolation branches 103 supply power to the first voltage transformation device 203 after passing through the high-frequency conversion module 202, that is, it cannot be ensured that the charging capacitor 231 releases the shock wave energy according to the preset high frequency, which improves the stability of the charging system to a certain extent.

In some possible embodiments, the charging control module 100 further comprises a first charging isolation circuit 101 connected in series with the power controller 102, and the first charging isolation circuit 101 is configured to receive an initial charging modulation signal and transmit the initial charging modulation signal to the power controller 102

Specifically, the first charging isolation circuit 101 is configured to isolate the initial charging modulation signal from the power controller 102, avoid potential safety hazards caconfigured by charging the charging capacitor 231 without receiving the initial charging modulation signal, and also avoid the influence of the peak voltage on the initial charging modulation signal during high-voltage operation, so that the initial charging modulation signal cannot drive the power controller 102 to work due to too small current.

Further, the first charging isolation circuit 101 comprises an optocoupler, and the first charging isolation circuit 101 is configured to isolate the initial charging modulation signal sent by the microcontroller from the charging control signal at the rear end, to avoid interference of the peak voltage on the microcontroller during high-voltage operation; if the peak voltage during high-voltage operation causes interference to the microcontroller, the microcontroller is damaged, resulting in an excessively small initial charging modulation signal generated by the microcontroller, and finally, the initial charging modulation signal cannot drive the power controller 102 to operate.

In some possible embodiments, the charging control module 100 further comprises a voltage collection circuit, the voltage collection circuit is configured to be electrically connected to the charging capacitor 231, and the voltage collection circuit is configured to detect an energy storage voltage of the charging capacitor 231 and output a voltage feedback signal based on the energy storage voltage.

Specifically, the voltage acquisition circuit is further electrically connected to the first charging isolation circuit 101, and when the voltage of the charging capacitor 231 is within a normal operation range, the first charging isolation circuit 101 is controlled to receive the initial charging modulation signal and transmit the initial charging modulation signal to the power controller 102; when the voltage of the charging capacitor 231 is in an abnormal operation range, the first charging isolation circuit 101 is controlled not to receive the initial charging modulation signal, and at this time, subsequent high-frequency charging of the charging capacitor 231 cannot be performed, thereby avoiding a potential safety hazard caconfigured by a failure of the charging capacitor 231 and charging.

In some embodiments, the charging power supply module 200 further comprises an isolation power supply 201, a charging power supply 205, an isolation power supply 201, and a high-frequency conversion module 202, which are sequentially connected in series.

In some embodiments, the charging power supply 205 is 220V alternating current, and the charging power supply module 200 further comprises a third transformer; and the third transformer is configured to convert an alternating current of the charging power supply 205 into a direct current to act on the isolation power supply 201, and a voltage of the direct current may be 90-360 V.

### Embodiment 2

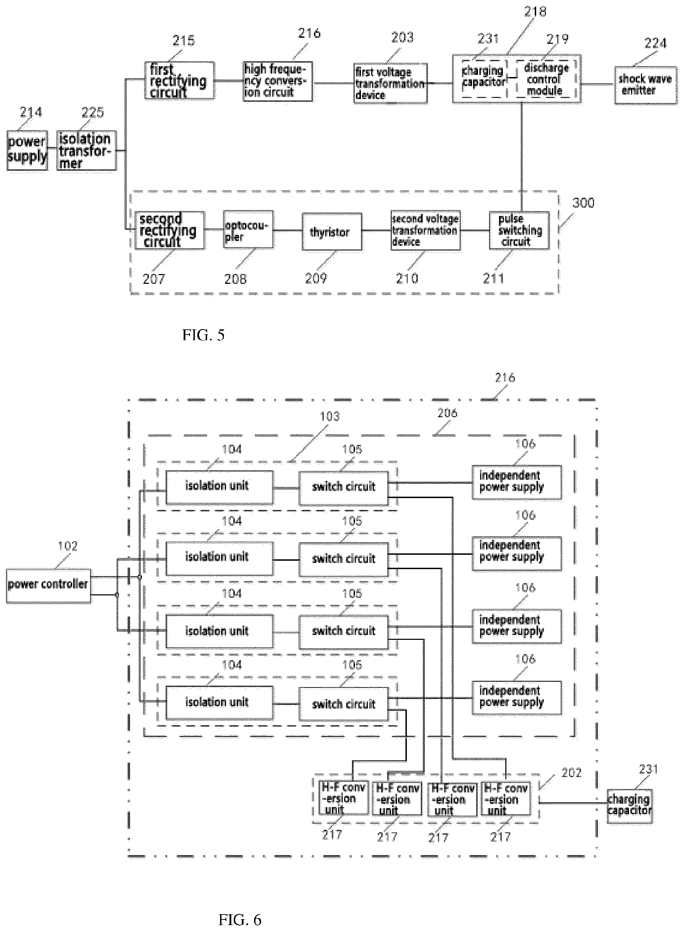

FIG. 5 is a structural diagram of an electrical isolation system according to an embodiment of this application, and the following describes the technical solution of this application in detail with reference to FIG. 5.

An embodiment of this application provides an electrical isolation system, applied to a shock wave device, wherein the electrical isolation system specifically comprises a signal trigger module 300, a first rectifying circuit 215, a high-frequency conversion circuit 216, and a discharge energy storage module 218 that are sequentially connected in series.

The input terminal of the first rectifying circuit 215 is connected to the power supply 214, and is configured to convert the alternating current into a direct current, and transmit the direct current to the high-frequency conversion circuit 216; the high frequency (HF) conversion circuit 216 comprises a high frequency (HF) conversion module 202 and a first isolation module 206; the high-frequency conversion module 202 is configured to convert the DC current into a high-frequency current, and transmit a high-frequency current to the discharge energy storage module 218; the high-frequency conversion module 202 comprises a plurality of high-frequency conversion units 217, the first isolation module 206 comprises N electrical isolation branches 103, and the high-frequency conversion units 217 are connected to the power controller 102 through the electrical isolation branch 103; wherein, N is an even number greater than zero; an output end of the discharge energy storage module 218 is connected to the shock wave emitter 224, an output end of the signal trigger module 300 is connected to the discharge energy storage module 218, and the signal trigger module 300 is configured to control the discharge energy storage module 218 to charge the shock wave emitter 224, and it should be noted that the frequency of the high-frequency current is 20 KHz -100 KHz.

In an embodiment, the current at the input end of the first rectifying circuit 215 is an alternating current of 110V -440 V, preferably 150 V -300 V, and exemplarily, the current is an alternating current of 170 V, 200V or 220V, and the first rectifying circuit 215 converts the alternating current into a direct current for charging the discharging energy storage module 218.

High-frequency charging is performed on the direct current output by the first rectifying circuit 215 through the high-frequency conversion circuit 216, so as to perform high-frequency charging on the shock wave generator with a high working voltage, for example, the normal working voltage of the shock wave generator may be greater than or equal to 10 kV, and therefore, high-frequency current is required to charge the shock wave generator to ensure normal operation of the shock wave generator.

Specifically, before the discharge energy storage module 218 supplies power to the shock wave emitter 224, the discharge energy storage module 218 needs to be charged, and in the process of charging the discharge energy storage module 218, the high-frequency conversion unit 217 controls on-off of the high-frequency conversion unit 217 through the electrical isolation branch 103, so as to change the leakage current of the surface of the shock wave generator with the working voltage greater than or equal to 10 kV, so as to ensure that the shock wave emitter 224 can be directly applied to the heart, greatly improve the dielectric strength of the electrical isolation system, ensure the safety of the whole electrical isolation system at a high operating voltage, and ensure that the shock wave transmitter 224 does not affect the human body.

It should be noted that the high-frequency conversion unit 217 has a good insulation effect, so that interference between the high-frequency conversion circuit 216 and the discharge energy storage module 218 can be effectively avoided, thereby reducing the leakage current of the electrical isolation system.

It should be noted that when the discharge energy storage module 218 receives the trigger signal, the discharge energy storage module 218 is turned on with the shock wave transmitter 224, and the discharge energy storage module 218 starts to supply power to the shock wave transmitter 224.

In an optional embodiment, the electrical isolation system may further comprise a first voltage transformation device 203, the high-frequency conversion circuit 216 is connected to the discharge energy storage module 218 through the first voltage transformation device 203, and the first voltage transformation device 203 is configured to boost the high-frequency current to ensure power supply to the discharge energy storage module 218.

In this embodiment of this application, by providing the first rectifying circuit 215, the high-frequency conversion circuit 216, and the discharge energy storage module 218, the charging speed of the discharge energy storage module 218 is improved, meanwhile, the leakage current of the shock wave emitter 224 in the normal working state is controlled to be 0.002 mA to 0.006 mA, and the dielectric strength of the electrical isolation system reaches 15 kV, so as to meet the safety performance requirements allowed by the shock wave generator with the electrical safety classification as the CF type.

In an optional embodiment, one electrical isolation branch 103 is connected to at least one high-frequency conversion unit 217, and preferably, one electrical isolation branch 103 is connected to one high-frequency conversion unit 217.

In this embodiment of this application, the power controller 102 may enable the at least one high-frequency conversion unit 217 to be in an on or off state through one electrical isolation branch 103, and the high-frequency conversion unit 217 in the on state converts the current into a high-frequency current, or the plurality of high-frequency conversion units 217 in the on state cooperatively convert the direct current into a high-frequency current, for example, as shown in FIG. 6, the power controller 102 may turn on or turn off a high-frequency conversion unit 217 through one electrical isolation branch 103.

In an actual application, the power controller 102 controls to charge the discharge energy storage module 218 at a desired charging frequency, specifically, the power controller 102 controls at least one high-frequency conversion unit 217 in the high-frequency conversion circuit 216 to be in an on state, thereby controlling the charging frequency of the high-frequency conversion circuit 216. The higher the conduction number of the high-frequency conversion unit 217 is proportional to the highest charging frequency that can be reached, that is, the more the high-frequency conversion units 217 are turned on, the higher the highest charging frequency for charging the discharge energy storage module 218. For example, when the conduction number of the high-frequency conversion unit 217 is 4, the charging frequency may reach 30 KHz, and when the conduction number of the high-frequency conversion unit 217 is 6, the charging frequency may reach 50 KHz, and so on. In a specific embodiment, the number of turns on the high-frequency conversion unit 217 may be controlled to be 4, 6, or 8, which is not specifically limited herein.

In an optional embodiment, N is an even number greater than 2 and less than or equal to 12, preferably, N is 4, 6 or 8, the number of the electrical isolation branches 103 is set to be greater than 2 and less than or equal to 12, on one hand, it is ensured that the electrical isolation branch 103 can effectively control the high-frequency conversion unit 217, thereby preventing one of the electrical isolation branches 103 from failing and affecting the overall charging efficiency; on the other hand, high-frequency charging is performed on the discharge energy storage module 218, so that the discharge energy storage module 218 provides electric energy to the shock wave emitter 224, thereby ensuring normal operation of the shock wave generator and realizing high-frequency charging. The number of the electrical isolation branches 103 may be 4, 6, or 8. In practical applications, as shown in FIG. 6, the number of the electrical isolation branches 103 may be set to 4, and when the number of the electrical isolation branches 103 is set to 4, the leakage current of the entire electrical isolation system may be controlled within a safe range, that is, the leakage current of the shock wave transmitter 224 in the normal working state is controlled to be 0.002 mA -0.006 mA, so that the dielectric strength of the entire electrical isolation system may reach 15 kV.

In a specific embodiment, the N electrical isolation branches 103 are divided into a plurality of groups, and each group of electrical isolation branches 103 is connected to the power controller 102, so as to receive a control signal sent by the power controller 102, and at the same time, avoid the influence between the electrical isolation branches 103, thereby improving the leakage current of the electrical isolation system. It should be noted that each group comprises at least two electrical isolation branches 103, so as to circularly control the conduction state between the electrical isolation branch 103 and the high-frequency conversion module 202, to implement high-frequency charging of the discharge energy storage module 218, thereby improving the charging speed of the discharge energy storage module 218.

In an optional embodiment, the first isolation module 206 comprises N independent power supplies 106, wherein the N independent power supplies 106 are connected to the N electrical isolation branches 103 in a one-to-one correspondence.

In this embodiment of this application, the independent power supply 106 may be a DC-DC power supply, wherein the independent power supply 106 and the power supply 214 are relatively independent, and the independent power supply 106 is configured to supply power to each electrical isolation branch 103 separately, so that the leakage current released by the first isolation module 206 can be effectively reduced to ensure effective control of the high-frequency conversion unit 217, and if one independent power supply is configured to supply power to the plurality of first isolation modules 206 at the same time, the stability of the electrical isolation system in the charging process cannot be ensured, and the safety performance when the shock wave transmitter 224 is configured to release the shock waves is reduced.

In a specific embodiment, the independent power supply 106 and the electrical isolation branch 103 may be disconnected or turned on, and the power controller 102 controls the independent power supply 106 and the electrical isolation branch 103 to be turned on or off, so that the high-frequency conversion unit 217 connected to the electrical isolation branch 103 is in an on state.

In another specific embodiment, the independent power supply 106 and the electrical isolation branch 103 are always in an on state, the electrical isolation branch 103 is in an off state or an on state, and the electrical isolation branch 103 enables the switch circuit 105 in the electrical isolation branch 103 to be in an on or off state based on the received control signal sent by the power controller 102, so that the high-frequency conversion unit 217 connected to the electrical isolation branch 103 is in an on or off state.

In an optional embodiment, the electrical isolation branch 103 further comprises an isolation unit 104 and a switch circuit 105.

The switch circuit 105 is connected to the power controller 102 through the isolation unit 104, a power connection end of the switch circuit 105 is connected to the independent power supply 106, an output end of the switch circuit 105 is connected to the high-frequency conversion unit 217, and the switch circuit 105 is configured to control, based on the received control signal sent by the power controller 102, the high-frequency conversion unit 217 to be turned on or turned off.

In this embodiment of this application, the isolation unit 104 is configured to isolate the power controller 102 from the switch circuit 105, thereby enhancing an isolation effect between the power controller 102 and the switch circuit 105, effectively reducing a leakage current in the electrical isolation system, and when the switch circuit 105 receives a control signal sent by the power controller 102, controlling the high-frequency conversion unit 217 to be turned on or off.

In an embodiment, the first rectifying circuit 215 and the second rectifying circuit 207 are separately powered to avoid interference between the first rectifying circuit 215 and the second rectifying circuit 207 to affect the safety of the entire electrical isolation system. For example, when the isolation transformer 225 is not configured, the leakage current generated when the shock wave emitter 224 releases the shock wave is reduced to about 30-50µA, the leakage current isolated by the isolation transformer 225 can be reduced to 3-6µA, thereby significantly reducing the leakage current when the shock wave emitter 224 releases the shock wave, and increasing the safety performance of using the shock wave transmitter 224.

The preset trigger signal received by the signal trigger module 300 may be a pulse signal, and when the preset trigger signal is a pulse signal, the requirement for precision is relatively high and easily interfered, so that the isolation transformer 225 needs to be isolated to further improve the dielectric strength of the electrical isolation system, thereby reducing the leakage current on the surface of the shock wave transmitter 224 and ensuring the safety performance of the shock wave transmitter 224.

In an optional embodiment, the signal trigger module 300 comprises an optocoupler 208 and a pulse switch circuit 211.

The optocoupler 208 is connected to the pulse switch circuit 211, the pulse switch circuit 211 is connected to the discharge energy storage module 218, and the optocoupler 208 is configured to control the pulse switch circuit 211 to be turned on based on the received preset trigger signal, so that the discharge energy storage module 218 provides electric energy to the shock wave transmitter 224.

Specifically, the pulse switch circuit 211 is connected to the discharge energy storage module 218, and when the pulse switch circuit 211 is turned on, the discharge energy storage module 218 is conducted with the shock wave emitter 224, so that the discharge energy storage module 218 provides electric energy to the shock wave transmitter 224.

In another embodiment, the signal trigger module 300 further comprises a second rectifying circuit 207, a thyristor 209, and a second voltage transformation device 210, where the optocoupler 208 is connected to the power supply 214 through the second rectifying circuit 207, and the optocoupler 208 is sequentially connected to the thyristor 209, the second voltage transformation device 210, and the pulse switch circuit 211.

In this embodiment of this application, the second rectifying circuit 207 is configured to convert the alternating current output by the power supply 214 into a direct current circuit, so as to provide energy for the optical coupler 208, and when the optical coupler 208 receives the preset trigger signal, the optical coupler 208 is turned on, so that the thyristor 209 is in an on state, the pulse switch circuit 211 is turned on, and the discharge energy storage module 218 is in communication with the shock wave transmitter 224, so that the discharge energy storage module 218 supplies power to the shock wave transmitter 224.

In an optional embodiment, the discharge energy storage module 218 comprises a charging capacitor 231 and a discharge control module 219, wherein a charging end of the charging capacitor 231 is connected to the high-frequency conversion circuit 216, and a discharge end of the charging capacitor 231 is connected to the shock wave transmitter 224 through a discharge control module 219.

In an optional embodiment, the shock wave emitter 224 is further comprised, an input end of the shock wave emitter 224 is connected to an output end of the shock wave generator, a plurality of electrodes 228 in the shock wave emitter 224 are connected to the discharge control module 219, and the discharge control module 219 is configured to control the shock wave emitter 224 to generate a shock wave.

Specifically, the discharge control module 219 is configured to control the shock wave emitter 224 to supply power, so that at least one electrode pair in the shock wave emitter 224 releases shock wave energy, wherein the electrode pair is composed of at least two electrodes 228, so as to form shock waves with different energy values, so that the release position and the treatment mode of the shock wave can be accurately controlled, different treatment modes are configured for different regions, and the treatment effect is improved.

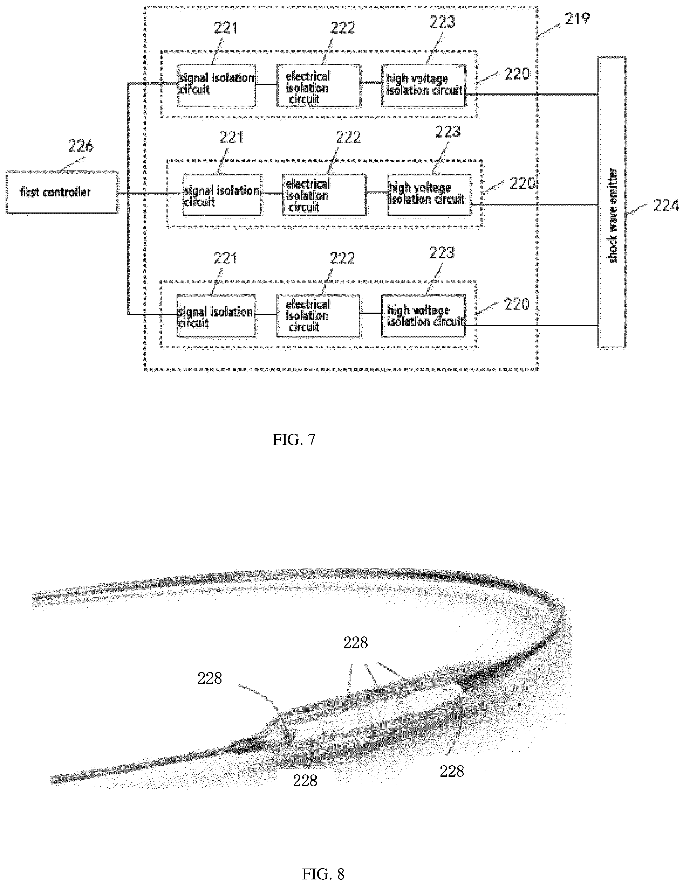

In an embodiment, as shown in FIG. 7, which is a structural diagram of a discharge control module according to an embodiment of this application, the discharge control module 219 comprises at least two discharge control units 220, the at least two discharge control units 220 are connected in parallel, and specifically, the discharge control unit 220 comprises a signal isolation circuit 221, an electrical isolation circuit 222, and a high-voltage isolation circuit 223, wherein the signal isolation circuit 221, the electrical isolation circuit 222, and the high-voltage isolation circuit 223 are sequentially connected in series.

In this embodiment of this application, an input end of the discharge control unit 220 is connected to the first controller 226, where the first controller 226 is an MCU controller, so as to receive a charging control signal sent by the first controller 226, and the discharge control unit 220 controls the shock wave emitter 224 to generate a shock wave based on the charging control signal.

Specifically, the signal isolation circuit 221 may comprise, but is not limited to, at least one of an optocoupler, a diode, a triode, and a low-voltage relay, and is configured to implement isolation between the first controller 226 and the electrical isolation circuit 222 by disposing the signal isolation circuit 221 between the first controller 226 and the electrical isolation circuit 222, to further reduce a leakage current of the entire electrical isolation system, and also improve stability of an output signal of the first controller 226.

The electrical isolation circuit 222 may comprise, but is not limited to, at least one of a diode, a high-voltage relay, a thyristor, and a field effect transistor, the electrical isolation circuit 222 has a function for enhancing isolation, and specifically, the signal isolation circuit 221 and the high-voltage isolation circuit 223 are isolated to further reduce the leakage current of the entire electrical isolation system and improve the stability of the entire electrical isolation system.

The high-voltage isolation circuit 223 comprises a high-voltage relay, and since the output end of the high-voltage isolation circuit 223 is connected to the shock wave transmitter 224, the high-voltage isolation circuit 223 is configured to isolate the shock wave generator and the charging capacitor 231 of which the discharge voltage is above 10 kV, thereby reducing the interference of the spike signal generated by the shock wave generator during high-voltage operation to the electrical isolation system as much as possible, and ensuring the normal operation of the electrical isolation system.

It should be noted that the shock wave generated by the shock wave emitter 224 may comprise, but is not limited to, a treatment applied to cardiac valve calcification and a treatment applied to endovascular calcification.

In an actual application, the discharge control module 219 may control the electrode 228 in the shock wave emitter 224 to release energy, so that the electrode 228 releases higher energy at a position closer to the lesion to improve the therapeutic effect; for example, when the shock wave generated by the shock wave emitter 224 is configured to treat the heart valve calcification, the discharge control module 219 controls the electrode 228 closest to the calcification position to release the shock wave energy, so that the electrode 228 releases higher energy at a position closer to the calcification position, thereby improving the treatment effect on heart valve calcification; the discharge control module 219 may further control the plurality of electrode pairs at different positions to release the shock wave energy, for example, the shock wave generated by the shock wave emitter 224 is applied to the treatment of endovascular calcification, as shown in FIG. 8, which is the structure diagram of the shock wave transmitter provided by the embodiment of the present application, and when the energy is concentrated to the target electrode pair in the shock wave emitter 224 through the discharge control module 219 to release, the target electrode in the shock wave emitter 224 can be treated when the catheter does not pass through the calcified region, and when the discharge control module 219 concentrates the energy into the target electrode pair in the shock wave emitter 224 to be released, the shock wave energy can be released to the periphery for treatment.

In an optional embodiment, the input terminal of the signal trigger module 300 is connected to the power supply 214, so that the power supply 214 provides energy to the signal trigger module 300. Specifically, the electrical isolation system further comprises an isolation transformer 225, an input end of the isolation transformer 225 is connected to the power supply 214, an output end of the isolation transformer 225 is respectively connected to an input end of the first rectifying circuit 215 and an input end of the signal trigger module 300, the isolation transformer 225 is configured to convert an alternating current output by the power supply 214 into an alternating current with a voltage value ranging from 150 V to 240 V and an alternating current with a voltage value ranging from 9 V to 24 V, wherein the alternating current with the voltage value ranging from 150 V to 240 V is configured to provide energy to the first rectifying circuit 215, and the alternating current with a voltage value ranging from 9 V to 24 V is configured to provide energy to the signal trigger module 300 to ensure normal operation of the first rectifying circuit 215 and the signal trigger module 300.

It can be seen from the above technical solutions of the embodiments of the present application that the charging process of the shock wave generator is realized by providing the first rectification circuit, the high-frequency conversion circuit and the discharge circuit which are sequentially connected in series, specifically, the input end of the first rectification circuit is connected to the power supply, and is configured to convert the alternating current into a direct current and deliver the direct current to the high-frequency conversion circuit; the high-frequency conversion module comprises a plurality of high-frequency conversion units, the first isolation module comprises N first isolation units, and each of the high-frequency conversion units is connected to the first controller through the first isolation unit; N is an even number greater than 2; and an output end of the discharge circuit is connected to the shock wave generator. The technical solution provided by the present application can greatly improve the dielectric strength of the electrical isolation system, reduce the leakage current on the surface of the shock wave generator, and ensure the safety performance of the shock wave generator.

### Embodiment 3

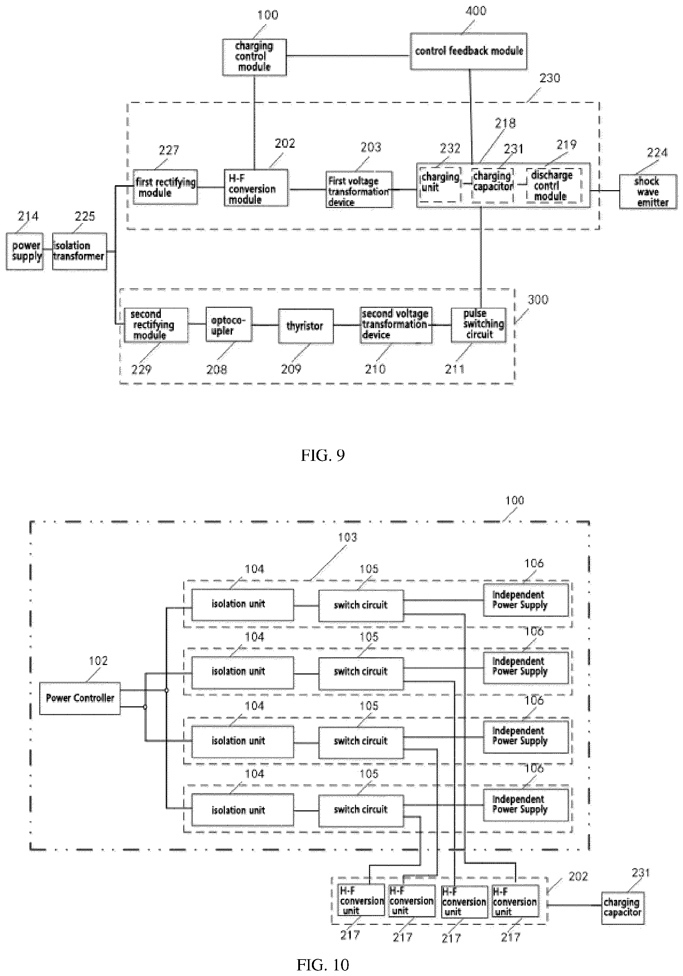

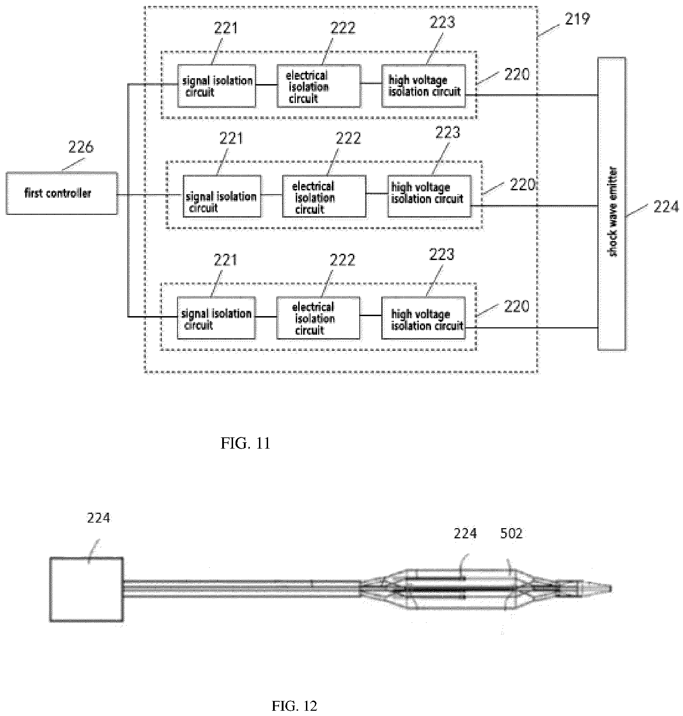

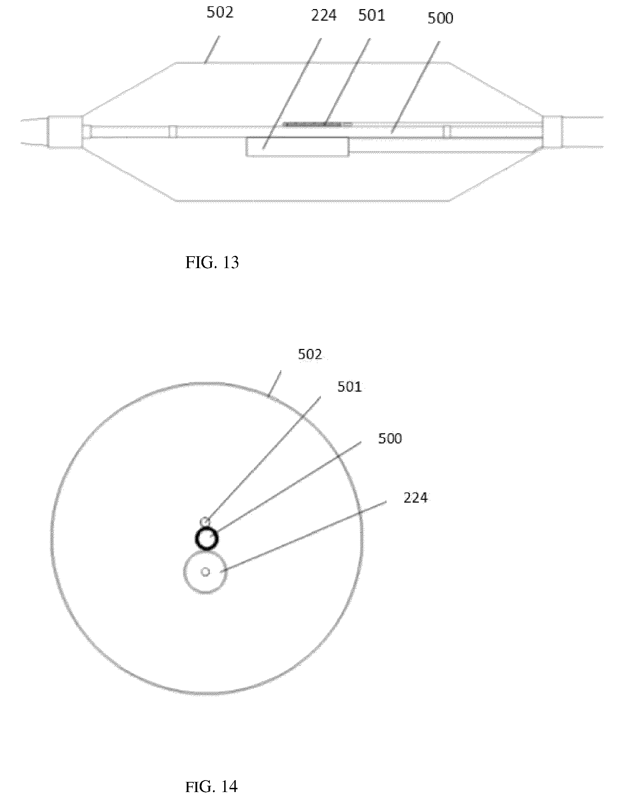

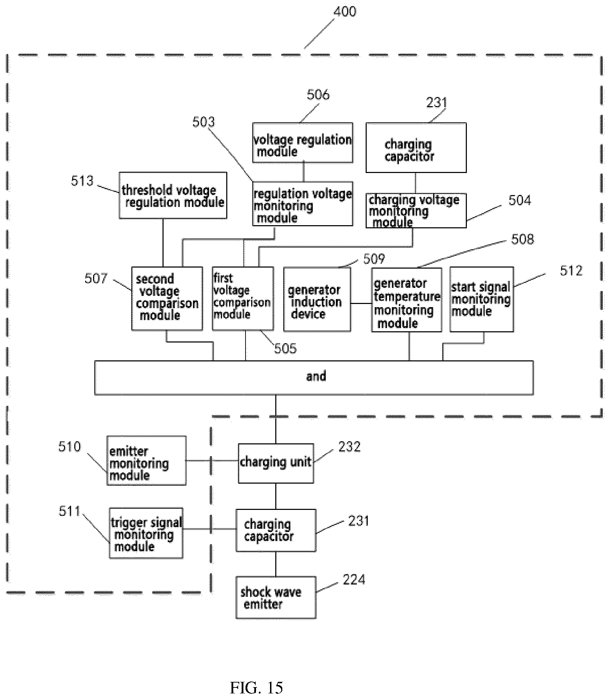

Referring to FIG. 9 to FIG. 15, the following describes the technical solutions of this application in detail with reference to FIG. 9 to FIG. 15.

An embodiment of this application provides a control system for a shock wave device, comprising: a signal triggering module 300, a charging control module 100, a charging module 230, and a control feedback module 400, wherein the charging control module 100 is electrically connected to the charging module 230; the charging module 230 comprises a high-frequency conversion module 202 and a discharge energy storage module 218 connected in series, and the control feedback module 400 is electrically connected to the charging control module 100 and the discharge energy storage module 218, respectively; a signal input terminal of the high-frequency conversion module 202 is electrically connected to an output terminal of the charging control module 100; a power input end of the high-frequency conversion module 202 is electrically connected to the power supply 214, an output end of the high-frequency conversion module 202 is electrically connected to the discharge energy storage module 218, and the high-frequency conversion module 202 can charge the discharge energy storage module 218; an output end of the signal trigger module 300 is electrically connected to the discharge energy storage module 218, an output end of the discharge energy storage module 218 is electrically connected to the shock wave emitter 224, and a power supply trigger signal output by the signal trigger module 300 can be transmitted to the discharge energy storage module 218, so that the discharge energy storage module 218 and the shock wave emitter 224 are in an on state.

In this embodiment of this application, the signal trigger module 300 is configured to send a power supply trigger signal to the discharge energy storage module 218, so that the discharge energy storage module 218 may receive a preset trigger signal output by the power controller 102, and in a case that the signal trigger module 300 receives the preset trigger signal, the signal trigger module 300 generates a power supply trigger signal, and transmits the power supply trigger signal to the discharge energy storage module 218, so that the discharge energy storage module 218 is in an on state when receiving the power supply trigger signal, so that the discharge energy storage module 218 provides energy to the shock wave transmitter 224.

In an embodiment, the high-frequency conversion module 202 is configured to convert a direct current into a high-frequency current, and transmit a high-frequency current to the discharge energy storage module 218, wherein an output value of the high-frequency conversion module 202 is determined according to a target control signal output by the charging control module 100, so as to output a high-frequency current to the discharge energy storage module 218. Specifically, when the high-frequency conversion module 202 receives the charging control signal output by the charging control module 100, when the high-frequency conversion module 202 receives the charging control signal to be turned on, the high-frequency conversion module 202 performs frequency rising processing on the charging control signal to obtain at least two frequency-rising charging control sub-signals, and supplies power to the shock wave transmitter 224 through the discharging energy storage module 218 based on the charging control sub-signal, so that the shock wave generator operates at a high-voltage and high-frequency, and the shock wave transmitter 224 releases more shock wave energy in unit time.

Specifically, the high-frequency conversion module 202 comprises a plurality of high-frequency conversion units 217, and the high-frequency conversion units 217 are connected to the power controller 102 through electrical isolation circuits, wherein the power controller 102 controls the on-off of the high-frequency conversion unit 217 through the electrical isolation circuit, so as to change the charging frequency for charging the discharge energy storage module 218, thereby improving the charging speed of the discharge energy storage module 218.

Further, the control feedback module 400 is configured to monitor and feed back the energy output of the shock wave transmitter 224, so as to control the charging unit 232 to supply power to the shock wave transmitter 224 through the charging capacitor 231 under the condition that the current discharge of all the shock wave emitters 224 is valid, so as to control the charging state of the shock wave generator in time, thereby significantly improving the safety and controllability of the system.

Meanwhile, by setting the signal triggering module 300, the charging control module 100, the charging module 230 and the control feedback module 400, the process of safe charging of the shock wave generator is realized, so that the charging frequency and the dielectric strength of the shock wave device can be greatly improved, the leakage current of the shock wave device is reduced, and the safety of treatment is further improved.

In an optional embodiment, the charging module 230 comprises a first voltage transformation device 203, the high-frequency conversion module 202 is connected to the discharge energy storage module 218 through the first voltage transformation device 203, and an input end voltage of the first voltage transformation device 203 is less than an output end voltage of the first voltage transformation device 203.

In an embodiment, the charging module 230 further comprises a first rectification module 227, wherein an output end of the first rectification module 227 is connected to an input end of the high-frequency conversion module 202, that is, the first rectification circuit 227, the high-frequency conversion module 202, the first voltage transformation device 203, and the discharge energy storage module 218 are sequentially connected in series.

Specifically, by arranging the first rectification module 227, the high-frequency conversion module 202, the first voltage transformation device 203 and the discharge energy storage module 218, the charging speed of the discharge energy storage module 218 is increased, meanwhile, the leakage current of the shock wave emitter 224 in the normal working state is controlled to be 0.002 mA -0.006 mA, and the dielectric strength of the control system reaches 15 kV, so as to meet the safety performance requirements allowed by the shock wave generator with the electrical safety classification as the CF type.

In an embodiment, the current at the input end of the first rectification module 227 is an alternating current of 110V -440 V, preferably a current of 150V -300 V, and exemplarily, the current is an alternating current of 170 V, 200V or 220V, and the first rectification module 227 converts the alternating current into a direct current for charging the high-frequency conversion module 202.

In an optional embodiment, the charging control module 100 comprises a power controller 102 and at least two electrical isolation circuits connected in parallel; an input end of the power controller 102 can receive an initial charging modulation signal, an output end of the power controller 102 is connected to an input end of the electrical isolation circuit, and an output end of the electrical isolation circuit is electrically connected to a signal input end of the high-frequency conversion module 202.

In this embodiment of this application, the power controller 102 is configured to perform frequency raising processing on the initial charging modulation signal, wherein a signal frequency of the charging control signal is higher than an initial charging modulation signal.

In this embodiment, by providing at least two electrical isolation circuits, the frequency of sending the charging control signal by the charging control module 100 to the charging module 230 is improved, thereby improving the power supply frequency of the charging module 230 to the shock wave transmitter 224, and further improving the frequency of the shock wave generation. Specifically, the first-stage frequency amplification is performed on the initial charging modulation signal through the power controller 102 to obtain the charging control signal, and the charging control signal performs secondary frequency amplification on the initial charging modulation signal through the at least two electrical isolation circuits to obtain the target control signal, that is, multiple times of frequency increase are performed on the signal, so that the shock wave generator can be charged at a high frequency in this embodiment.

In an optional embodiment, each of the at least two electrical isolation circuits comprises two electrical isolation branches 103 connected in parallel, wherein the high-frequency conversion module 202 comprises at least four signal input ends, the electrical isolation branches 103 are arranged in one-to-one correspondence with the signal input ends, the leakage current in the charging process of the shock wave generator is effectively reduced through the control of the plurality of electrical isolation branches 103 on the high-frequency conversion module 202, so that the shock wave emitter 224 can be directly applied to the heart, the dielectric strength of the control system is greatly improved, the safety of the whole control system under a high working voltage is ensured, and it is ensured that the shock wave emitter 224 does not negatively affect the human body.

In an optional embodiment, the electrical isolation branch 103 comprises an isolation unit 104, a switch circuit 211, and an independent power supply 106; the switch circuit 211 is connected with the power controller 102 through the isolation unit 104, the power connection end of the switch circuit 211 is connected with the independent power supply 106, the output end of the switch circuit 211 is connected with the signal input end of the high-frequency conversion module 202, the leakage current released by the isolation unit 104 can be effectively reduced, and effective control over the high-frequency conversion module 202 is ensured; if one independent power supply 106 is configured to supply power to the multi-channel electrical isolation branch 103 at the same time, the leakage current of the electrical isolation circuit can be greatly increased, so that the stability of the charging system in the charging process cannot be ensured, and the safety performance when the shock wave transmitter 224 releases the shock wave is affected.

Specifically, the switch circuit 211 is configured to isolate the isolation unit 104 from the independent power supply 106, and when a fault occurs in the isolation unit 104, cut off the current switch circuit 211 in time to avoid affecting the independent power supply 106 or other electrical isolation branches 103; when the independent power supply 106 fails, the current switch circuit 211 is cut off in time to avoid affecting the isolation unit 104 or other electrical isolation branches 103; meanwhile, each of the electrical isolation branches 103 is provided with the independent power supply 106 to ensure that the electrical isolation branches 103 do not affect each other, ensure the operation stability of the charging system, and the switch circuit 211 is also configured to electrically isolate the electrical isolation branch 103 from the back-end circuit, thereby avoiding the interference of the high-voltage high-frequency to the front end, and further ensuring the operation stability of the charging system.

For example, when the two parallel electrical isolation branches 103 in the electrical isolation circuit are turned on under the control of the switching circuit 211, the corresponding one of the signal receiving circuits in the high-frequency conversion module 202 electrically connected to the two parallel electrical isolation branches 103 is turned on to generate a target control signal to directly control charging; and then the on or off of the signal receiving circuit in the high-frequency conversion module 202 can be controlled by turning on or off the switching circuit 211, thereby adjusting the charging frequency of the shock wave generator.

Further, the isolation unit 104 comprises an optocoupler 208, a MOS transistor and a triode, the power supply 214 configured by the component is an independent power supply 106, the output signal of the power generator is electrically isolated from the rear end of the power generator through the optocoupler 208, the MOS transistor and the triode, interference of the high-voltage high-frequency part to the front end is avoided, and the operation stability of the charging system is improved.

In an optional embodiment, the signal trigger module 300 comprises an optocoupler 208 and a pulse switch circuit 211, wherein the optocoupler 208 is connected in series with the pulse switch circuit 211, and the pulse switch circuit 211 is connected to the discharge energy storage module 218.

In this embodiment of this application, the optocoupler 208 is configured to control the pulse switch circuit 211 to be turned on based on the received preset trigger signal, so that the discharge energy storage module 218 provides electric energy to the shock wave transmitter 224.

Specifically, the pulse switch circuit 211 is connected to the discharge energy storage module 218, and when the pulse switch circuit 211 is turned on, the discharge energy storage module 218 is conducted with the shock wave emitter 224, so that the discharge energy storage module 218 provides electric energy to the shock wave transmitter 224.

In another embodiment, the signal trigger module 300 further comprises a second rectification module 229, a thyristor 209, and a second voltage transformation device 210, wherein the optocoupler 208 is connected to the power supply 214 through the second rectification module 229, and the optocoupler 208 is sequentially connected to the thyristor 209, the second voltage transformation device 210, and the pulse switch circuit 211.

In this embodiment of this application, the second rectification module 229 is configured to convert the alternating current output by the power supply 214 into a direct current circuit, so as to provide energy for the optical coupler 208, and when the optical coupler 208 receives the preset trigger signal, the optical coupler 208 is turned on, so that the thyristor 209 is in an on state, the pulse switch circuit 211 is turned on, and the discharge energy storage module 218 is in communication with the shock wave transmitter 224, so that the discharge energy storage module 218 supplies power to the shock wave transmitter 224.

It should be noted that the first rectification module 227 and the second rectification module 229 are separately powered to avoid interference between the first rectification module 227 and the second rectification module 229 to affect the safety of the entire electrical isolation system. Specifically, the first rectification module 227 is configured to convert the AC current into a DC current, and then increase the frequency and voltage of the DC current to charge the discharge energy storage module 218. For example, when the isolation transformer 225 is not configured, the leakage current generated when the shock wave emitter 224 releases the shock wave is about 30-50 µA, the leakage current isolated by the isolation transformer 225 can be reduced to 3-6 µA, thereby significantly reducing the leakage current when the shock wave emitter 224 releases the shock wave, and increasing the safety performance of using the shock wave transmitter 224.

In an optional embodiment, the discharge energy storage module 218 comprises a charging unit 232, a charging capacitor 231 and a discharge control module 219, wherein an input end of the charging unit 232 is electrically connected to the high-frequency conversion module 202, an output end of the charging unit 232 is electrically connected to a charging end of the charging capacitor 231, and a discharge end of the charging capacitor 231 is electrically connected to the shock wave transmitter 224 through a discharge control module 219.

Specifically, the discharge control module 219 is configured to control the shock wave emitter 224 to charge, so that at least one electrode pair in the shock wave emitter 224 releases the shock wave energy, wherein the electrode pair is composed of at least two electrodes to form shock waves with different energy values, so that the release position and the treatment mode of the shock wave can be accurately controlled, different treatment modes are configured for different regions, and the treatment effect is improved.

In an embodiment, the discharge control module 219 comprises at least two discharge control units, the at least two discharge control units are connected in parallel, and specifically, the discharge control unit comprises a signal isolation circuit 221, an electrical isolation sub-circuit 234 and a high-voltage isolation circuit 223, wherein the signal isolation circuit 221, the electrical isolation sub-circuit 234 and the high-voltage isolation circuit 223 are sequentially connected in series.

In this embodiment of this application, an input end of the discharge control unit is connected to the first controller 226, where the first controller 226 is an MCU controller, so as to receive a charging control signal sent by the first controller 226, and the discharge control unit controls the shock wave emitter 224 to generate a shock wave based on the charging control signal.

Specifically, the signal isolation circuit 221 may comprise, but is not limited to, at least one of an optocoupler 208, a diode, a triode, and a low-voltage relay, and is configured to implement isolation between the first controller 226 and the electrical isolation sub-circuit 234 by disposing the signal isolation circuit 221 between the first controller 226 and the electrical isolation sub-circuit 234, to further reduce a leakage current of the entire electrical isolation system, and also improve stability of an output signal of the first controller 226.

The electrical isolation sub-circuit 234 may comprise, but is not limited to, at least one of a diode, a high-voltage relay, a thyristor 209, and a field effect transistor, the electrical isolation sub-circuit 234 has a function for enhancing isolation, and specifically, the signal isolation circuit 221 and the high-voltage isolation circuit 223 are isolated to further reduce the leakage current of the entire electrical isolation system and improve the stability of the entire electrical isolation system.

The high-voltage isolation circuit 223 comprises a high-voltage relay, and since the output end of the high-voltage isolation circuit 223 is connected to the shock wave transmitter 224, the high-voltage isolation circuit 223 is configured to isolate the shock wave generator and the charging capacitor 231 of which the discharge voltage is above 10 kV, thereby reducing the interference of the spike signal generated by the shock wave generator during high-voltage operation to the electrical isolation system as much as possible, and ensuring the normal operation of the electrical isolation system.

It should be noted that the shock wave generated by the shock wave emitter 224 may comprise, but is not limited to, a treatment applied to cardiac valve calcification and a treatment applied to endovascular calcification.

In this embodiment of this application, the transmitter sensing apparatus 501 is configured to collect a target state parameter of the shock wave transmitter 224 in a working state, where there is a preset correspondence between the target state parameter and the discharge energy of the shock wave transmitter 224; and the transmitter monitoring module 510 is configured to receive the target state parameter transmitted by the transmitter sensing apparatus 501, and output a negative feedback signal to the shock wave generator when the target state parameter does not meet the preset discharge condition, where the negative feedback signal is configured to indicate that the shock wave generator stops charging.

Specifically, in the discharging process of the shock wave transmitter 224, the target state parameter of the shock wave transmitter 224 is configured to indicate the magnitude of the discharge energy of the shock wave transmitter 224 in the current working state, and the transmitter sensing device 501 corresponding to the shock wave transmitter 224 collects the target state parameter generated by each discharge of the shock wave transmitter 224 and transmits the target state parameter to the transmitter monitoring module 510; if the target state parameter does not meet the preset discharge condition, it indicates that the current discharge of the shock wave transmitter 224 is invalid, and if the current discharge of any shock wave transmitter 224 is invalid, the transmitter monitoring module 510 transmits a negative feedback signal to the shock wave generator, and the shock wave generator stops charging in response to the negative feedback signal; if the target state parameter meets the preset discharge condition, it indicates that the current discharge of the shock wave emitter 224 is valid, and in the case that the current discharge of all the shock wave emitters 224 is valid, the transmitter monitoring module 510 transmits a positive feedback signal to the shock wave generator, and charges in response to the positive feedback signal. Specifically, the preset discharge condition may be that each parameter in the target state parameter is within a preset range of each parameter.

In one embodiment, where the shock wave transmitter 224 is discharging inactive, indicating that the control system is abnormal, the shock wave generator is controlled to stop operating. Optionally, in a case that discharge of the shock wave transmitter 224 is invalid, fault warning information is generated and displayed. Specifically, the fault information is displayed by the display device of the shock wave generator, or the fault light of the shock wave generator flickers.

In this application, the transmitter sensing device 501 is disposed at the shock wave transmitter 224 and electrically connected to the transmitter monitoring module 510, so that the target state parameter of the shock wave transmitter 224 in the working state can be monitored in real time, and whether the negative feedback signal is output to the shock wave generator or not is judged based on the target state parameter and the preset discharge condition, so that real-time monitoring and feedback of the energy output of the shock wave generator are realized, and the charging state of the shock wave generator is controlled in time, so that the system safety and controllability are remarkably improved.

In an optional embodiment, the device further comprises a balloon 502 and a positioning inner tube 500, the balloon 502 is internally provided with a shock wave emitter 224 and a transmitter sensing device 501, the positioning inner tube 500 extends into the balloon 502, the shock wave emitter 224 is fixedly connected to a partial conduit extending into the balloon 502 by the positioning inner tube 500, and the transmitter sensing device 501 is fixedly connected to a partial conduit extending into the balloon 502 by the positioning inner tube 500.

The shock wave emitter 224 and the positioning inner tube 500 extend into a fixed connection manner of a partial conduit of the balloon 502, and the transmitter sensing device 501 and the positioning inner tube 500 extend into a fixed connection manner of a partial conduit of the balloon 502. In the axial direction of the positioning inner tube 500, the distance between the detection point of the emitter sensing device 501 and the discharge point of the shock wave emitter 224 is 4 mm. In this way, the target state parameter generated by the shock wave transmitter 224 can be accurately collected, and the damage of the high-energy shock wave to the transmitter sensing device 501 can also be prevented, thereby prolonging the service life of the transmitter sensing device 501.

In an optional embodiment, the control feedback module 400 further comprises a voltage adjustment module 506, an adjustment voltage monitoring module 503, a charging voltage monitoring module 504, a first voltage comparison module 505, and a second voltage comparison module 507

The first voltage comparison module 505 is electrically connected to the adjustment voltage monitoring module 503 and the charging voltage monitoring module 504, respectively, the adjustment voltage monitoring module 503 is electrically connected to the voltage adjustment module 506, the charging voltage monitoring module 504 is connected to the charging capacitor 231, and the second voltage comparison module 507 is electrically connected to the adjustment voltage monitoring module 503.

Specifically, the adjustment voltage monitoring module 503 is configured to detect a voltage setting signal output by the voltage adjustment module 506, and transmit the voltage setting signal to the first voltage comparison module 505; The charging voltage monitoring module 504 is configured to detect a current voltage signal of the charging capacitor 231 and transmit the current voltage signal to the first voltage comparison module 505; the first voltage comparison module 505 is configured to compare the voltage setting signal with the current voltage signal to generate a voltage comparison feedback signal, where the voltage comparison feedback signal is configured to indicate an on-off state between the charging unit 232 and the charging capacitor 231; and the second voltage comparison module 507 is configured to receive the voltage setting signal transmitted by the adjustment voltage monitoring module 503 and compare the voltage setting signal with the output voltage threshold to generate an output voltage feedback signal, where the output voltage feedback signal is configured to indicate an on-off state between the charging unit 232 and the charging capacitor 231.

Further, the control feedback module 400 further comprises a threshold voltage regulation module 513, a threshold voltage regulation module 513, and a second voltage comparison module 507. Specifically, in response to a control signal sent by the shock wave generator, the threshold voltage adjustment module 513 adjusts and outputs an output voltage threshold.

Further, the second voltage comparison module 507 receives the voltage setting signal transmitted by the regulation voltage monitoring module 503 and the output voltage threshold transmitted by the threshold voltage regulation module 513, and when the voltage setting signal is less than the output voltage threshold, the second voltage comparison module 507 generates a first output voltage feedback signal, where the first output voltage feedback signal is configured to instruct the charging unit 232 to charge the charging capacitor 231; When the voltage setting signal is greater than or equal to the output voltage threshold, the second voltage comparison module 507 generates a second output voltage feedback signal, and the second output voltage feedback signal is configured to instruct the charging unit 232 to stop charging the charging capacitor 231. In this way, the charging and discharging safety of the control system can be further ensured.

In this embodiment of this application, the control feedback module 400 further comprises a trigger signal monitoring module 511, where the trigger signal monitoring module 511 is configured to generate a trigger feedback signal based on a trigger signal without a receive generator, and the trigger feedback signal is configured to indicate a working state of the charging capacitor 231.

Further, in response to the closing of the foot switch or the handle switch, the second control module of the shock wave generator generates a generator trigger signal. In a case that the trigger signal monitoring module 511 receives the generator trigger signal, the trigger signal monitoring module 511 generates a first trigger feedback signal, where the first trigger feedback signal is configured to instruct the charging capacitor 231 to discharge; and when the trigger signal monitoring module 511 does not receive the generator trigger signal, the trigger signal monitoring module 511 generates a second trigger feedback signal, wherein the second trigger feedback signal is configured to instruct the charging capacitor 231 to stop discharging.

In an optional embodiment, the control feedback module 400 further comprises a temperature monitoring module 508 and a sensing device 509 disposed on the shock wave transmitter 224, wherein the sensing device 509 is electrically connected to the temperature monitoring module 508, and specifically, the sensing device 509 is configured to collect an operating temperature of a target component in the shock wave transmitter 224 and transmit the operating temperature to the temperature monitoring module 508; the temperature monitoring module 508 is configured to generate a temperature feedback signal based on the working temperature and a preset working temperature threshold, wherein the temperature feedback signal is configured to indicate an on-off state between the charging unit 232 and the charging capacitor 231.

Further, when the working temperature of the target component is less than or equal to the preset working temperature threshold, the temperature monitoring module 508 generates a temperature positive feedback signal, and the temperature positive feedback signal is configured to instruct the charging unit 232 to charge the charging capacitor 231; and when the working temperature of the target component is greater than the preset working temperature threshold, the temperature monitoring module 508 generates a temperature negative feedback signal, and the temperature negative feedback signal is configured to instruct the charging unit 232 to stop charging the charging capacitor 231.

In an optional embodiment, the control feedback module 400 further comprises: a start signal monitoring module 512, where the start signal monitoring module 512 is configured to receive a generator start signal, generate a start feedback signal when the generator start signal is received, and start the feedback signal to indicate an on-off state between the charging unit 232 and the charging capacitor 231.

Specifically, when the transmitter monitoring module 510 generates a generator positive feedback signal, the first voltage comparison module 505 generates a first voltage comparison positive feedback signal, the second voltage comparison module 507 generates a first output voltage feedback signal, and when the temperature monitoring module 508 generates a temperature positive feedback signal and the start signal monitoring module 512 generates a generator start signal, the charging unit 232 charges the charging capacitor 231, otherwise, the charging unit 232 stops charging the charging capacitor 231, thereby not only increasing the circuit safety of the shock wave system, but also improving the therapeutic effect of the shock wave system.

It can be seen from the above technical solutions of the embodiments of the present application that the signal trigger module, the charging control module, the charging module and the control feedback module are provided to safely charge the shock wave device, specifically, the charging control module is electrically connected to the charging module; The charging module comprises a high-frequency conversion module and a discharge energy storage module connected in series, and the control feedback module is electrically connected to the charging control module and the discharge energy storage module, respectively; A signal input end of the high-frequency conversion module is electrically connected to an output end of the charging control module; a power input end of the high-frequency conversion module is electrically connected to the power supply, and an output end of the high-frequency conversion module is electrically connected to the discharge energy storage module; an output end of the signal trigger module is electrically connected to the charging module, an output end of the charging module is electrically connected to the shock wave generator, and a charging trigger signal output by the signal trigger module can be transmitted to the charging module, so that the charging module and the shock wave generator are in an on state. The technical solution provided by the present application can greatly improve the charging frequency and dielectric strength of the shock wave device, reduce the leakage current of the shock wave device, and further improve the safety of treatment.

This application further provides a shock wave device, comprising a shock wave transmitter and the control system as described above, and details are not described herein again.

It should be noted that the shock wave generated by the shock wave device may comprise, but is not limited to, a treatment applied to cardiac valve calcification and a treatment applied to endovascular calcification.

Although the present invention has been described by the preferred embodiments, it is not intended that the invention be limited to the embodiments described herein, but that various changes and modifications may be made without departing from the scope of the invention.

The foregoing descriptions are merely specific embodiments of this application, but the protection scope of this application is not limited thereto, and any changes or substitutions may be easily conceived of by a person skilled in the art within the technical scope disclosed in this application. Therefore, the protection scope of this application shall be subject to the protection scope of the claims.

## Claims

1. A charging system, comprising a charging control module and a charging power supply module which are connected in parallel;
the charging control module comprises a power controller and at least two parallel electrical isolation circuits; the charging power supply module comprises a high-frequency conversion module and a rectification module which are connected in series;
an input end of the power controller is configured to receive an initial charging modulation signal, an output end of the power controller is electrically connected to an input end of the electrical isolation circuit, and an output end of the electrical isolation circuit is electrically connected to a signal input end of the high-frequency conversion module;
a power input terminal of the high-frequency conversion module is electrically connected to a charging power supply, and an output terminal of the high-frequency conversion module is electrically connected to the rectification module;
the initial charging modulation signal generates a charging control signal after passing through the power controller, and the charging control signal is transmitted to the high-frequency conversion module through at least two electrical isolation circuits, and the high-frequency conversion module is turned on in response to the charging control signal to charge a charging capacitor of the discharging energy storage module.

2. The charging system according to claim 1, wherein the power controller is configured to perform frequency raising processing on the initial charging modulation signal, and a signal frequency of the charging control signal is higher than the initial charging modulation signal.

3. The charging system according to claim 1, wherein the charging control signal comprises at least two charging control sub-signals of different phases, and at least two of the charging control sub-signals are respectively transmitted to the high-frequency conversion module through the at least two electrical isolation circuits;
the high-frequency conversion module is configured to perform frequency raising processing on the at least two charging control sub-signals to output at least two target control signals, wherein a signal frequency of the target control signal is higher than the charging control sub-signal.

4. The charging system according to claim 3, wherein each of the at least two electrical isolation circuits comprises two electrical isolation branches connected in parallel;
the high-frequency conversion module comprises at least four signal input ends, and the electrical isolation branches are provided in one-to-one correspondence with the signal input ends.

5. The charging system according to claim 4, wherein the electrical isolation branch comprises an isolation unit, a switch circuit and an independent power supply, an input end of the isolation unit is electrically connected to the power controller, and the switch circuit is electrically connected to the isolation unit, the independent power supply, and the high-frequency conversion module, respectively.

6. The charging system according to any one of claims 1-5, wherein the charging power supply module further comprises a first voltage transformation device, the first voltage transformation device is connected in series with the high-frequency conversion module and the rectification module, and an input end voltage of the first voltage transformation device is less than an output end voltage of the first voltage transformation device.

7. The charging system according to any one of claims 1-5, wherein the charging control module further comprises a first charging isolation circuit connected in series with the power controller, and the first charging isolation circuit is configured to receive the initial charging modulation signal and transmit the initial charging modulation signal to the power controller.

8. The charging system according to any one of claims 1-5, wherein the charging control module further comprises a voltage acquisition circuit;
the voltage acquisition circuit is configured to be electrically connected to the charging capacitor, and the voltage acquisition circuit is configured to detect an energy storage voltage of the charging capacitor and output a voltage feedback signal based on the energy storage voltage.

9. The charging system according to any one of claims 1-5, wherein the charging power supply module further comprises an isolation power supply, and the charging power supply, the isolation power supply, and the high-frequency conversion module are sequentially connected in series.

10. An electrical isolation system applied to a shock wave device, wherein the electrical isolation system comprises a signal trigger module, a first rectification module, a high-frequency conversion circuit and a discharge energy storage module which are sequentially connected in series;
an input terminal of the first rectification module is connected to a power supply to convert an AC current into a DC current, and transmit the DC current to the high-frequency conversion circuit; and
the high-frequency conversion circuit comprises a high-frequency conversion module and an electrical isolation branch; the high-frequency conversion module is configured to convert the direct current into a high-frequency current, and transmit the high-frequency current to the discharge energy storage module; the high-frequency conversion module comprises a plurality of high-frequency conversion units, the electrical isolation branch comprises N isolation units, and the high-frequency conversion units are connected to the first controller through the isolation unit; wherein N is an even number greater than 2;
an output terminal of the discharge energy storage module is connected to the shock wave transmitter;
an output end of the signal trigger module is connected to the discharge energy storage module, and the signal trigger module is configured to control the discharge energy storage module to supply power to the shock wave transmitter.

11. The electrical isolation system according to claim 10, wherein one of the isolation units is connected to at least one of the high-frequency conversion units, and preferably, one of the isolation units is connected to one of the high-frequency conversion units.

12. The electrical isolation system according to claim 11, wherein the electrical isolation branch comprises N independent power supplies;
the N independent power sources are connected to the N isolation units in a one-to-one correspondence.

13. The electrical isolation system according to claim 10, wherein the signal trigger module comprises an optocoupler and a pulse switch circuit;
the optocoupler is connected to the pulse switch circuit, the pulse switch circuit is connected to the discharge energy storage module, and the optocoupler controls the pulse switch circuit to be turned on based on a received preset trigger signal, so that the discharge energy storage module provides electric energy to the shock wave transmitter.

14. The electrical isolation system according to claim 10, wherein the discharge energy storage module comprises a charging capacitor and a discharge control module;
a charging end of the charging capacitor is connected to the high-frequency conversion circuit, and a discharging end of the charging capacitor is connected to the shock wave transmitter through the discharge control module.

15. A control system, applied to a shock wave device, comprising: a signal triggering module, a charging control module, a charging module and a control feedback module;
the charging control module is electrically connected to the charging module;
the charging module comprises a high-frequency conversion module and a discharge energy storage module which are connected in series, and a signal input end of the high-frequency conversion module is electrically connected to an output end of the charging control module;
the control feedback module is electrically connected to the charging control module and the discharge energy storage module, respectively;
a power input end of the high-frequency conversion module is electrically connected to a power supply, an output end of the high-frequency conversion module is electrically connected to the discharge energy storage module, and the high-frequency conversion module charges the discharge energy storage module;
an output end of the signal trigger module is electrically connected to the discharge energy storage module, an output end of the discharge energy storage module is electrically connected to the shock wave transmitter, and a charging trigger signal output by the signal trigger module is transmitted to the discharge energy storage module, so that the discharge energy storage module and the shock wave transmitter are in an on state.

16. The control system according to claim 15, wherein the charging control module comprises a power controller and at least two electrical isolation circuits connected in parallel; an input end of the power controller receives an initial charging modulation signal, an output end of the power controller is connected to an input end of the electrical isolation circuit, and an output end of the electrical isolation circuit is electrically connected to a signal input end of the high-frequency conversion module.

17. The control system according to claim 16, wherein each of the at least two electrical isolation circuits comprises two electrical isolation branches connected in parallel;
the high-frequency conversion module comprises at least four signal input ends, and the electrical isolation branches are provided in one-to-one correspondence with the signal input ends.

18. The control system according to claim 15, wherein the charging module comprises a first voltage transformation device, the high-frequency conversion module is connected to the discharge energy storage module through the first voltage transformation device, and an input end voltage of the first voltage transformation device is less than an output end voltage of the first voltage transformation device.

19. The control system according to claim 17, wherein the electrical isolation branch comprises an isolation unit, a switch circuit and an independent power supply;
the switch circuit is connected to the power controller through the isolation unit, a power connection end of the switch circuit is connected to the independent power supply, and an output end of the switch circuit is connected to a signal input end of the high-frequency conversion module.

20. The control system according to claim 15, wherein the discharge energy storage module comprises a charging unit, a charging capacitor and a discharge control module;
an input end of the charging unit is electrically connected to the high-frequency conversion module, an output end of the charging unit is electrically connected to a charging end of the charging capacitor, and a discharging end of the charging capacitor is electrically connected to the shock wave generator through the discharge control module.

21. The control system according to claim 15, wherein the control feedback module comprises a transmitter sensing device, a transmitter monitoring module and a trigger signal monitoring module;
the transmitter sensing device is provided opposite to the shock wave transmitter, the shock wave transmitter is electrically connected to the shock wave generator, and the transmitter monitoring module is electrically connected to the transmitter sensing device and the discharge energy storage module, respectively;
the trigger signal monitoring module is electrically connected to the charging capacitor.

22. The control system according to claim 15, wherein the control feedback module further comprises a voltage adjustment module, an adjustment voltage monitoring module, a charging voltage monitoring module and a first voltage comparison module, and the first voltage comparison module is electrically connected to the adjustment voltage monitoring module and the charging voltage monitoring module, respectively;
the adjustment voltage monitoring module is configured to detect a voltage setting signal output by the voltage adjustment module, and transmit the voltage setting signal to the first voltage comparison module; the charging voltage monitoring module is configured to detect a current voltage signal of the charging capacitor and transmit the current voltage signal to the first voltage comparison module; and the first voltage comparison module is configured to compare the voltage setting signal with the current voltage signal to generate a voltage comparison feedback signal, wherein the voltage comparison feedback signal is configured to indicate an on-off state between the charging unit and the charging capacitor.

23. The control system according to claim 22, wherein the control feedback module further comprises a second voltage comparison module, and the second voltage comparison module is electrically connected to the adjustment voltage monitoring module;
the second voltage comparison module is configured to receive the voltage setting signal transmitted by the adjustment voltage monitoring module, and compare the voltage setting signal with an output voltage threshold to generate an output voltage feedback signal, wherein the output voltage feedback signal is configured to indicate an on-off state between the charging unit and the charging capacitor.

24. The control system according to claim 22, wherein the control feedback module further comprises a temperature monitoring module and a sensing device provided on the shock wave generator, and the sensing device is electrically connected to the temperature monitoring module;
the sensing device is configured to collect a working temperature of a target component in the shock wave generator, and transmit the working temperature to the temperature monitoring module; and
the temperature monitoring module is configured to generate a temperature feedback signal based on the working temperature and a preset working temperature threshold, wherein the temperature feedback signal is configured to indicate an on-off state between the charging unit and the charging capacitor.

25. A shock wave device, comprising a shock wave transmitter and the control system according to any one of claims 15-24.
